# EUROPEAN PATENT APPLICATION

(11) **EP 2 050 757 A1**
(43) Date of publication of application: **22.04.2009**
(21) Application number: 07019826.2
(22) Date of filing: 10.10.2007
(51) Int. Cl.: C07H 19/12

(54) **Method of producing 2' -deoxy-5-azacytidine (Decitabine)**

(71) Applicant: Cilag AG, 8205 Schaffhausen (CH)
(72) Inventor: Kraut, Norbert., 78250 Tengen (DE); Jungmann, Oliver., 78166 Donaueschingen (DE)
(74) Representative: Braun, André jr.

(57) **Abstract**

Summary

Method of producing 2'-deoxy-5-azacytidine (Decitabine) by providing a compound of formula (I): 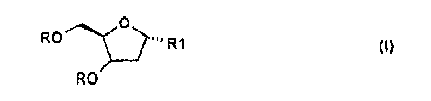 wherein
R is a removable substituent known per se; and R₁ is a glycoside donor preferably a 1-halogen derivative, an imidate preferably the trichloromethyl derivative, or a thio-alkyl derivative; further providing a silylated
base of formula (II): 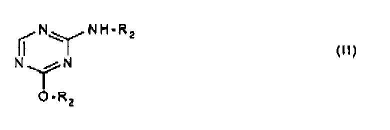 wherein R₂ is a protecting group, preferably a trimethylsilyl TMS)-residue; reacting the compound of formula (I) and the compound of formula (II) together in a suitable anhydrous solvent and in the presence of a suitable catalyst; and removing the substituents R from the compound obtained in order to obtain the compound 2'-deoxy-5-azacytidine (Decitabine), characterized in that said catalyst is selected from the group comprising a salt of an aliphatic sulphonic acid and/or the salt of a optionally substituted aromatic sulphonic acid.

## Description

The present invention refers to a method of producing 2'-deoxy-5-azacytidine (Decitabine) by reacting a glycoside donor preferably a 1-halogen derivative, or an imidate preferably a trichloromethyl derivative, or a thio-alkyl derivative of a blocked monosaccharide with a selected silylated base in the presence of a selected catalyst.

Decitabine is a nucleoside and a known pharmaceutically active compound. From US 3,817,980 it is known to synthesize nucleosides by silylating the corresponding nucleoside base and reacting the silylated base with a glycosyl donor preferably a 1-halogen derivative of a blocked monosaccharide or oligosaccharide in the presence of a selected catalyst by silylating the base and reacting the sugar derivative in a single step in the presence of trimethylhalosilane and/or hexamethyldisilazane. The catalysts used are e.g. selected from SnCl₄, TiCl₄, ZnCl₂, BF₃-etherate, AlCl₃ and SbCl₅, or a trialkylester of a perfluoroalkanesulfonic acid. The major disadvantage is that all catalysts mentioned in the patent (tin tetrachloride, titanium tetrachloride, zinc chloride or boron trifluoride etherate) are prone to hydrolysis giving irritant hydrolysis products like HCl or are forming insoluble oxides (TiO₂, SnO₂), which are difficult to remove from the reaction product and difficult to handle, especially on large scale production.

It has now been found that a 1-halo sugar derivative can be reacted with a silylated or alkylated 5-azacytosine in the presence of a selected catalysts, i.e. an inorganic salt like lithium trifluoromethane sulfonate, to give excellent product yields. This type of catalyst is stable under aqueous conditions, easy to handle, does not produce irritant hydrolysis products, and can be easily removed. Instead of the highly toxic and cancerogenious solvent dichloroethane as exemplified in US 3,817,980, much less toxic solvents such as dichloromethane can be used. Additionally, the selectivity of the reaction for obtaining the desired anomer, i.e. ratio of the alpha/beta anomers, and the final yields are considerably improved. Using the reaction conditions of the present invention a selectivity of at least 1:2 in favor of the beta-isomer (β-isomer), in the average about three quarter of the reaction yield is the beta isomer and, depending on the particular reaction condition, a ratio of the alpha to the beta isomer of 18:82 was obtained. Further, according to the present invention a reaction yield that is higher than 95% (reaction yield >95%), and regularly is within the range of 97-98% is obtained.

The present invention is defined in the claims. The present invention refers to a method of producing 2'-deoxy-5-azacytidine (Decitabine) by providing a compound (a blocked monosaccharide derivative) of formula (I); 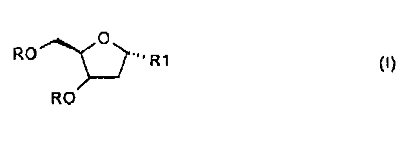 wherein
R is a removable substituent (protecting group) known per se, preferably (C₁-C₈)alkylcarbonyl, or optionally substituted phenylcarbonyl, or optionally substituted benzylcarbonyl;
R1 a glycoside donor preferably a 1-halogen derivative, or an imidate preferably a trichloromethyl derivative, or a thio-alkyl derivative;
further providing a silylated base of formula (II): 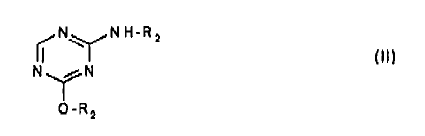 wherein R₂ is a protecting group, preferably a trimethylsilyl (TMS)-residue;
reacting the compound of formula (I) and the compound of formula (II) together in a suitable anhydrous solvent and in the presence of a suitable catalyst, whereby the compound of formula (III): 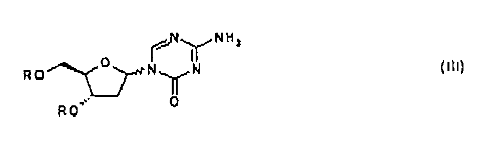 is obtained; and removing the substituent R in order to obtain the compound 2'-deoxy-5-azacytidine (Decitabine), characterized in that said catalyst is selected from the group comprising a salt of an aliphatic sulphonic acid and/or the salt of a optionally substituted aromatic sulphonic acid.

The present invention refers also to the production of the compound of formula (III). Preferred is the beta-glycoside, as indicated in formula (III).

The catalyst used in said reaction preferably is a salt of an aliphatic sulphonic acid, preferably of methylsulphonic acid (mesylate) or of ethylsulphonic acid, or of a fluorinated aliphatic sulfonic acid, such as a salt of trifluoromethane-sulfonic acid, pentafluoroethyl-sulfonic acid, or heptafluoropropyl-sulfonic acid. Preferred of these salts are the salts of methylsulphonic acid (mesylate) and the salts of trifluoromethane-sulfonic acid. Preferred are the alkali salts, and earth alkali salts. Preferred further are the lithium salts, preferably lithium methylsulphonic acid (lithium mesylate) or lithium-trifluoromethanesulfonate (LiOTf, lithiumtriflate). Generally also other salts, for example the salts of scandium, such as Sc(OTf)₃ or of copper such as Cu(OTf)₂ or of magnesium such as Mg(OTf)₂ can be used. However, the lithium salt and especially LiOTf is preferred. Further salts of known, optionally substituted, aromatic sulphonic acids may also be used; preferably the alkali salts and earth alkali salts thereof. Preferred are the alkali salts and earth alkali salts of benzenesulphonic acid (besylate), toluenesulphonic acid (tosylate), and known related acids. Preferred are the lithium salts of said aromatic sulphonic acids.

Preferred solvents to carry out the reaction according to the present invention are chlorinated solvents, such as dichloromethane, dichloroethane, chloroform, chlorobenzene, or toluene, xylol, acetonitril and/or propylene carbonate or related solvents. Preferred are chlorinated solvents. Preferred is the use of lithium-trifluoromethanesulfonate (LiOTf) in a chlorinated solvent, preferably in dichloromethane, dichloroethane, chloroform and/or chlorobenzene.

The compound of formula (I) and its preparation are known per se. The removable substituent R is preferably (C₁-C₄)alkylcarbonyl, or optionally substituted phenylcarbonyl, like phenylcarbonyl, tolylcarbonyl, xylylcarbonyl or benzylcarbonyl; preferably acetyl or p-chloro-phenylcarbonyl. The removable substituent R₁ is preferably halogen, preferably chlorine, bromine, fluorine, preferably chlorine, or an imidate, preferably trichloromethyl imidate, or a thio-alkyl derivative, preferably -S-methyl.

The preparation of compound of formula (II) and its preparation are known. The compound is preferably prepared by reaction of the free base with trimethylchlorosilane or with hexamethyldisilazane.

When reacting the compounds of formulae (I) and (II) together, the reaction temperature generally is within the range of 0°C to about 90°C, preferably at about room temperature, whereby the components are reacted in about equimolar amounts and preferably with a slight excess of compound of formula (II). The catalyst is used preferably in a concentration of about 10 mol-% - 90 mol-%, calculated to the total molar presence of the two reacting components. For the expert in the art it is no problem to optimize the molar ratios of the components.

For removing the substituents R from the compound of formula (III) in order to obtain the compound 2'-deoxy-5-azacytidine (Decitabine), containing free hydroxyl groups, known methods are used. The substituents R may be preferably removed, for example, by treatment in an alcoholic solution of ammonia or alcoholates; but other known methods may be applied. The following example illustrates the invention.

### Example 1

(A) A mixture of 5-Azacytosine (20 g, 178.4 mmol), ammonium sulfate (2.4 g, 18.16 mmol), and hexamethyldisilazane (160 g, 991.3 mmol) was heated to reflux until a clear solution was obtained. The excess of hexamethyldisilazane was removed in the vacuum at 60°C.
(B) A mixture of 264 g of dichloromethane, lithium trifluoromethane sulfonate (12.53 g, 80.3 mmol) and the "chloro sugar" C-137 [69.0 g, 160.6 mmol, corresponding to compound of formula (I)] were prepared and added to the residue obtained in step (A).
(C) The mixture was stirred for 4 hours at ambient temperature (20-25°C). Then the solvent was removed at 40°C in the vacuum and the obtained residue was dissolved in 60 g ethyl acetate. The solution was added drop wise to a mixture of 220 g of aqueous sodium hydrogen carbonate (2.5% w-solution), 174 g ethyl acetate, 36 g cyclohexane and 70 g acetonitrile at 30°C and the obtained reaction mixture is cooled to 0°C and stirred for 3 h. The precipitate of the blocked (protected) aminotriazine was filtered off, washed with water and finally with a mixture of acetonitrile and ethyl acetate (1:1).
   Yield: 70.0 g; 60.6% [assay (beta-isomer): 70.3 %; purity (beta-isomer): 64.7%].
   Scheme 1 shows the chemical reaction.

### Example 2

The compound corresponding to formula (III) as obtained in Example 1 is further treated with in an alcoholic solution of ammonia in a known manner so that 2'-deoxy-5-azacytidine (Decitabine) is obtained in practically quantitative yield.

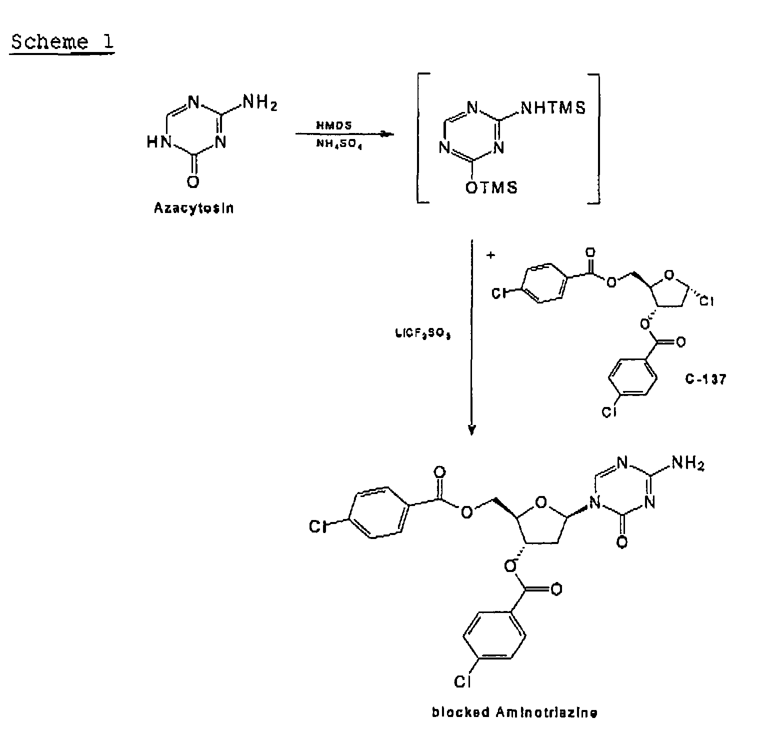

## Claims

1. Method of producing 2'-deoxy-5-azacytidine (Decitabine) by providing a compound of formula (I): 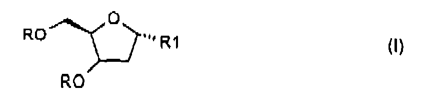 wherein
R is a removable substituent known per se, preferably (C₁-C₈)alkylcarbonyl, or optionally substituted phenylcarbonyl, or optionally substituted benzylcarbonyl;
R1 is a glycoside donor preferably a 1-halogen derivative, or an imidate preferably a trichloromethyl derivative, or a thio-alkyl derivative, preferably thiomethyl derivative; further providing a silylated base of formula (II): 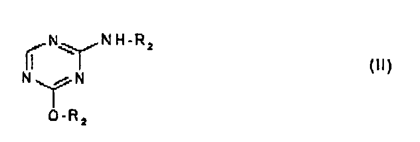 wherein R₂ is a protecting group, preferably a trimethylsilyl (TMS)-residue;
reacting the compound of formula (I) and the compound of formula (II) together in a suitable anhydrous solvent and in the presence of a suitable catalyst, whereby the compound of formula (III): 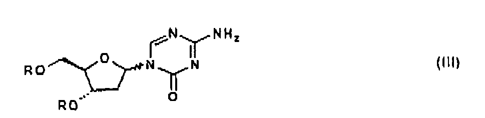 is obtained; and removing the substituents R in order to obtain the compound 2'-deoxy-5-azacytidine (Decitabine), **characterized in that** said catalyst is selected from the group comprising a salt of an aliphatic sulphonic acid and/or the salt of a optionally substituted aromatic sulphonic acid.

2. Method of producing a compound of formula (III) according
to claim 1, by providing a compound of formula (I): 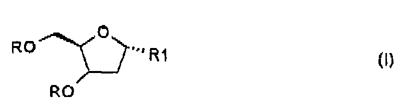 wherein
R is a removable substituent known per se, preferably (C₁-C₈)alkylcarbonyl, or optionally substituted phenylcarbonyl, or optionally substituted benzylcarbonyl;
R₁ is a glycoside donor preferably a 1-halogen derivative, or an imidate preferably a trichloromethyl derivative, or a thio-alkyl derivative preferably thiomethyl; further providing a silylated base of formula (II): 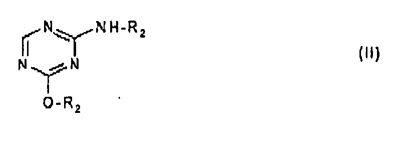 wherein R₂ is a protecting group, preferably a trimethylsilyl (TMS)-residue;
reacting the compound of formula (I) and the compound of formula (II) together in a suitable anhydrous solvent and in the presence of a suitable catalyst, whereby the compound of formula (III) is obtained, **characterized in that** said catalyst is selected from the group comprising a salt of an aliphatic sulphonic acid and/or the salt of a optionally substituted aromatic sulphonic acid.

3. Method according to claim 1 or 2, **characterized in that** the catalyst used in said reaction is a salt of an aliphatic sulphonic acid, preferably of methylsulphonic acid or of ethyl-sulphonic acid, or a salt of a fluorinated aliphatic sulfonic acid, preferably of trifluoromethane-sulfonic acid, pentafluoroethyl-sulfonic acid, or heptafluoropropyl-sulfonic acid.

4. Method according to claim 3, **characterized in that** the catalyst is a salt of methylsulphonic acid and/or the salts of trifluoromethanesulfonic acid.

5. Method according to any one of the claims 1-4, **characterrized in that** the catalyst is an alkali salt or earth alkali salt, preferably a lithium salt, preferably lithium methylsulphonic acid or lithium-trifluoromethanesulfonate.

6. Method according to claim 1 or 2, **characterized in that** the catalyst is chosen from the salts comprising salts of scandium preferably Sc(OTf)₃; or salts of copper preferably Cu(OTf)₂; or salts of magnesium preferably Mg(OTf)₂.

7. Method according to claim 1 or 2, **characterized in that** the catalyst is chosen from the salts comprising optionally substituted, aromatic sulphonic acids, preferably the alkali salts and earth alkali salts thereof.

8. Method according to claim 7, **characterized in that** the catalyst is chosen from the salts comprising alkali salts and earth alkali salts of benzenesulphonic acid, toluenesulphonic acid, and known related acids, preferably the lithium salt of said aromatic sulphonic acids.

9. Method according to any one of the claims 1-8, **characterrized in that** the solvent to carry out the reaction is chosen from the group comprising chlorinated solvents, preferably dichloromethane, dichloroethane, chloroform, chlorobenzene; or toluene, xylol, acetonitril and/or propylene carbonate or related solvents, and preferably is a chlorinated solvent.

10. Method according to any one of the claims 1-9, **characterrized in that** the catalyst is lithium-trifluoromethanesulfonate and the solvent is a chlorinated solvent, preferably dichloromethane, dichloroethane, chloroform and/or chlorobenzene.

11. Method according to any one of the claims 1-10, characterrized in that the removable substituent R is (C₁-C₄)alkyl-carboxyl, or optionally substituted phenylcarbonyl or benzylcarbonyl, preferably phenylcarbonyl, tolylcarbonyl, xylylcarbonyl; preferably acetyl or p-chloro-phenylcarbonyl.

12. Method according to any one of the claims 1-11, characterrized in that the removable substituent R1 is -O-acyl(C₁-C₄), -O-alkyl(C₁-C₄) or chlorine, preferably -O-(O)CCH₃ or chlorine, preferably chlorine.
